# EUROPÄISCHE PATENTSCHRIFT

(11) **EP 3 294 348 B1**
(45) Veröffentlichungstag und Bekanntmachung des Hinweises auf die Patenterteilung: **17.04.2019**
(21) Anmeldenummer: 16722828.7
(22) Anmeldetag: 03.05.2016
(51) Int. Cl.: A61L 9/03, B60H 3/00

(54) **BEDUFTUNGSGERÄT**
ELECTRIC FRAGRANCE DIFFUSER
DIFFUSEUR DE PARFUM

(30) Priorität: 09.05.2015 DE 102015006101
(43) Veröffentlichungstag der Anmeldung: 21.03.2018
(73) Patentinhaber: Grättinger, Günter, 82319 Starnberg (DE)
(72) Erfinder: ORTNER, Georg, verstorben (DE)
(86) Internationale Anmeldenummer: PCT/EP2016/059820
(87) Internationale Veröffentlichungsnummer: WO 2016/180663

(56) Entgegenhaltungen:
- FR-A1- 2 606 595
- US-A- 5 484 086
- US-A1- 2003 194 355
- US-A1- 2009 220 222
- US-A1- 2011 049 259
- US-A1- 2011 290 911
- US-A1- 2014 126 892

## Beschreibung

Die vorliegende Erfindung betrifft ein Beduftungsgerät für Innenräume, insbesondere Personenzellen in Kraftfahrzeugen, mit austauschbarem Duftstoffbehälter für flüssigen Duftstoff und einem elektrischen Heizelement zum Verdunsten von Duftstoff vor dessen Ausströmen aus dem Beduftungsgerät.

Derartige Beduftungsgeräte haben in der Kraftfahrzeugindustrie bereits Eingang gefunden, mit dem Ziel der Steigerung der Fahrsicherheit und des Wohlbefindens von Personen während der Fahrt.

Nicht nur für Automotive-Anwendungen geht es um die Beduftung von Innenräumen mit gleichbleibender Qualität des Dufterlebnisses und mit der Möglichkeit der individuellen Duftauswahl. Darüber hinaus geht es auch darum, unangenehme Düfte zu überdecken, wie beispielsweise den Nikotingeruch im Cockpit von Kraftfahrzeugen. Neben attraktiven Geruchserwartungen können auch therapeutische Zwecke erfüllt werden, beispielsweise durch Auswahl von erfrischenden Naturdüften, etwa wie Wald, Meeresbrise oder von spezifischen Düften nach ätherischen Ölen wie Orangenöl, Pfefferminzöl oder dergleichen.

Dabei ist es für das Dufterlebnis von besonderer Bedeutung, dass ein ausgewählter Duft gleichbleibend nachgeliefert wird, d.h. unerwünschte Entmischungen und unangenehme Veränderungen durch Oxidation vermieden werden.

US5,484,086 offenbart einen beheizten Bedufter mit Reservoir, aus welchem mittels einer Kapillare Duftstoff gesaugt und zu einer elektrischen Heizung transportiert wird.

Bei einem bekannten Duftspender für den Innenraum eines Fahrzeugs (DE 203 02 097 U1) ist eine Anzahl mehrerer Duftstoffbehälter vorgesehen, die mittels einer elektrisch gesteuerten Vorrichtung wahlweise ausgewählt werden können, um auf diese Weise Duftnoten zu wechseln. Ein elektrisches Heizelement sorgt dabei für die Verdunstung des Duftstoffs, dessen Austritt aus dem Duftstoffbehälter durch ein elektrisch ansteuerbares Ventil ermöglicht wird. Die elektrische Energie kann wahlweise über einen Adapter aus einem Zigarettenanzünder bezogen werden.

Ferner ist eine Beduftungseinrichtung für Kraftfahrzeuge bekannt (DE 10 2008 021 460 A1), welche in ein im Kraftfahrzeug vorhandenes Bauteil integriert ist, beispielsweise den Schalthebel, das Lenkrad oder den Fahrzeuginnenspiegel und welche einen hierfür geeigneten Mikroventilator zur Förderung der Duftstoffausbreitung aufweist. Die bekannte Beduftungseinrichtung umfasst darüber hinaus nachfüllbare oder austauschbare Duftstoffvorratsbehälter. Die Verdunstung erfolgt passiv, d.h. ohne gesonderte Erwärmung des Duftstoffs, z.B. durch bloße Nutzung der Körperwärme des Fahrers, der seine Hand auch während der Schaltpausen auf dem Schaltknauf belässt. Im Falle des Einbaus der Beduftungseinrichtung in das Lenkrad ist sie in einer Lenkradspeiche eingebaut, wobei die Betätigung des Lenkrads für eine passive Beduftung sorgt, oder es ist eine aktive Beduftung mit Hilfe eines kleinen Ventilators mit einer zum Fahrer hin gerichteten Luftaustrittsöffnung in der Lenkradspeiche vorgesehen.

Mittels eines bekannten Funktionsknopfs für einen Duftstoffbehälter (WO 2012/104351 A1) eines Beduftungssystems in einem Kraftfahrzeug wird Luft über einen Einlasskanal in einen mit Duftstoff befüllten Duftstoffbehälter eingeleitet und die dadurch mit Duftmolekülen angereicherte Luft über einen Auslasskanal dem Innenraum des Kraftfahrzeugs zugeführt. Bei einer derartigen aktiven Strömungsführung der Luft besteht die Gefahr einer Oxidation des Duftstoffs mit der Folge einer raschen Qualitätsminderung desselben, so dass zur Abhilfe ein Austausch des Duftstoffs lange vor dem vollständigen Aufbrauchen des Duftstoffvorrats in Betracht kommt.

Demgegenüber liegt der vorliegenden Erfindung die Aufgabe zugrunde, bei einem Beduftungsgerät der eingangs genannten Art eine gleichbleibende Qualität des Duftstoffs zu gewährleisten, bis der gesamte Duftstoffvorrat aufgebraucht ist und ferner eine Entmischung des Duftstoffs durch vorzeitiges Entweichen besonders flüchtiger Bestandteile zu vermeiden, so dass nicht nur Duftstoffe mit hoher Stabilität gegenüber Luftsauerstoff in Frage kommen.

Diese Aufgabe wird erfindungsgemäß durch eine Vorrichtung gemäß Anspruch 1 gelöst.

Für den Betrieb elektrischer Geräteeinbauteile kann ein Gerätestecker zum Anschließen an eine externe Stromquelle vorgesehen sein.

Durch die erfindungsgemäße Nutzung der Kapillarkraft zur Entnahme des Duftstoffs aus dem Duftstoffbehälter und dessen Förderung mittels einer durch ein Heizelement unterstützten Luftkonvektion gelingt es ohne zusätzliche Energiezufuhr den Duftstoff zu entnehmen und nach außen zu verteilen. Durch die Nutzung der Kapillarkraft zur Aktivierung dieser Vorgänge kann auf separate Transportmittel für den Duftstoff verzichtet werden. Damit wird ein Beduftungsgerät in Miniaturbauweise verwirklicht, welches für die Versorgung kleiner Innenräume mit geringen Duftstoffmengen ausreicht.

Auch eine Belüftung des Inneren des Duftstoffbehälters zum Zwecke der Entnahme von Duftstoff entfällt vollständig, somit auch die schädliche Oxidation des Duftstoffs. Durch den kapillaren Duftstofftransport bleibt der Duftstoff in seiner Qualität und Frische bis zur vollständigen Entleerung des Duftstoffbehälters erhalten.

Für den Betrieb des erfindungsgemäßen Beduftungsgeräts ist eine elektronische Regeleinrichtung vorteilhaft, die mit einem Gerätestecker, bevorzugt einem USB-Gerätestecker zum Anschließen an eine externe Stromquelle verbunden ist. Die Regeleinrichtung sorgt für die Vorgabe der Ein- und Auszeiten des Gerätebetriebs einschließlich des Heizelements. Dessen Aufgabe besteht im Wesentlichen darin, einen im Beduftungsgerät geführten schwachen Luftstrom durch Konvektion zu erzeugen. Dieser erwärmte Luftstrom sorgt für die Verdunstung der durch Kapillarkraft dem Duftstoffbehälter entnommenen flüssigen Duftstoffmenge und für die Verteilung des bedufteten Luftstroms.

Dabei kann die Stromversorgung über den USB-Stecker wahlweise über den Zigarettenanzünder im Auto, am Arbeitsplatz über einen Computer oder über eine sonstige externe Stromquelle erfolgen. Vorteilhaft kann auch eine spezielle Andockstation benutzt werden.

Eine geeignete Andockstation ist bevorzugt derart ausgebildet, dass ihre Unterseite eine Standfläche bildet, dass auf ihrer Oberseite ein USB-Geräteeingang vorgesehen ist und dass sie einen USB-Anschluss für eine externe Stromquelle, z.B. den Computer aufweist.

Zusätzlich kann im Inneren der Andockstation ein Ventilator eingebaut sein, der an eine Ausblasöffnung auf ihrer Oberseite angeschlossen ist und auf diese Weise zur aktiveren Verteilung der mit Duftstoff angereicherten Luft, die aus dem Beduftungsgerät ausströmt, sorgt.

Die Nutzung der Kapillarkraft kann erfindungsgemäß in der Weise erfolgen, dass wenigstens eine mit einem Ansaugende in den Duftstoffbehälter eintauchende Kapillarröhre vorgesehen ist, deren abgewandtes Austrittsende zwecks Verdunstung des austretenden Duftstoffs belüftet ist. Die Kapillarröhre taucht dabei möglichst tief in den Duftstoffbehälter ein, so dass dessen vollständige Entleerung sichergestellt ist. Je nach Auslegung des Beduftungsgeräts kann auch eine Mehrzahl von Kapillarröhren vorgesehen sein.

Um eine möglichst effiziente Einwirkung des Heizelements auf den aus der Kapillarröhre austretenden Duftstoff zu gewährleisten, ist nach einer vorteilhaften Ausgestaltung vorgesehen, dass der Duftstoff unmittelbar im Anschluss an das Austrittsende der Kapillarröhre einer Verteilungseinrichtung zugeführt wird, die dem Heizelement zugeordnet ist. Auf diese Weise wird gleichzeitig erreicht, dass der Luftstrom nach dessen Erwärmung durch das Heizelement vom Duftstoffbehälter zumindest soweit ferngehalten wird, dass dessen Inhalt nicht einer direkten Belüftung ausgesetzt wird, so dass eine Oxidation des Duftstoffs unterbleibt.

Die Verteilungseinrichtung weist bevorzugt eine Lamellenanordnung auf, deren Lamellenzwischenräume zur Verteilung des Duftstoffs mittels Kapillarkraft geeignet ausgebildet sind.

Der aus der Kapillarröhre austretende Duftstoff gelangt dabei noch im flüssigen Zustand zur Verteilung zwischen den Lamellen, wobei er vom Öffnungsbereich des Duftstoffbehälters durch Kapillarwirkung weiterfließt, so dass er im Bereich der Lamellenanordnung vom Warmluftstrom erfasst wird und verdunstet.

Bei einer Variante der Erfindung ist eine aktive Belüftung des Duftstoffs, nach dem er den Duftstoffbehälter verlassen hat, in Form einer gesonderten Ventilatoreinrichtung vorgesehen, beispielsweise in Art eines elektrischen Mikroventilators. Dieser ist bevorzugt zur Erzeugung einer schwachen Luftströmung auf einer Seite des Heizelements angeordnet, auf dessen anderen Seite die Verteilungseinrichtung positioniert ist, also derart, dass sich das Heizelement zwischen Ventilatoreinrichtung und Verteilungseinrichtung befindet.

Die Bauteile des Beduftungsgeräts nämlich neben dem Duftstoffbehälter und der lamellenartigen Verteilungseinrichtung noch das Heizelement und die Ventilatoreinrichtung sind im Inneren eines im Wesentlichen geschlossenen Gehäuses eingebaut, welches auf einander gegenüberliegenden Seiten Öffnungen für den Eintritt von Umgebungsluft und für den Austritt von bedufteter Luft aufweist. Auf diese Weise wird die Entstehung eines für die Duftverteilung ausreichenden Luftstroms begünstigt, der das Gehäuse entweder getrieben durch Konvektion oder beschleunigt durch die Verteilungseinrichtung durchströmt, indem er zuerst im Wärmetausch mit dem Heizelement erwärmt wird und danach die Verteilungseinrichtung durchströmt, wobei der dort vorhandene flüssige Duftstoff verdunstet und in molekularer Form mit dem Luftstrom verteilt wird.

Eine besonders vorteilhafte Ausgestaltung der Erfindung besteht darin, dass Duftstoffbehälter mit Kapillarröhre und Verteilungseinrichtung zusammen als austauschbare Wechselkartusche ausgebildet sind. Damit wird sichergestellt, dass die Verteilungseinrichtung, wie immer sie ausgestaltet ist, beispielsweise in Form eines Lamellenpakets, wie oben beschrieben, stets nur mit demselben Duftstoff in Berührung kommt bzw. von diesem an der Oberfläche benetzt wird. Ein Austausch des Duftstoffs gegen einen anderen bedeutet damit stets auch den Austausch der Verteilungseinrichtung, so dass jede Vermengung verschiedener Duftstoffe unterbleibt. Durch einen Austausch einer Kartusche gegen eine andere kommt es also zwingend zu einem gemeinsamen Austausch von Duftstoffbehälter und Verteilungseinrichtung, bei Vermeidung einer nachteiligen Duftstoffkontamination indem ein neuer Duftstoff nicht über Oberflächen der Verteilungseinrichtung geleitet wird, die bereits durch einen anderen Duftstoff benetzt worden sind.

Zur Erleichterung des Kartuschenaustauschs ist vorgesehen, dass das Gehäuse auf seiner Seite benachbart der Ausströmöffnung des Duftstoffbehälters mittels einer abnehmbaren Kappe versehen ist. Nach dem Abnehmen der Kappe kann die Kartusche mit dem aufgebrauchten Duftstoff entnommen und durch eine neue Kartusche entweder mit dem gleichen Duftstoff oder mit einem anderen Duftstoff ersetzt werden.

Der der elektrischen Versorgung des Beduftungsgeräts dienende USB-Gerätestecker ist vorteilhaft am Gehäuse auf dessen der Kappe gegenüberliegenden Seite nach außen vorspringend angebaut, so dass das Beduftungsgerät in jeden im Innenraum des Kraftfahrzeugs vorhandenen USB-Anschluss eingesteckt werden kann, bevorzugt allerdings derart, dass der Duftstoffbehälter senkrecht positioniert ist. Diese Position gewährleistet z.B. der genannte Adapter für den Zigarettenanzünder oder die genannte Andockstation.

Im Folgenden wird ein Ausführungsbeispiel der Erfindung anhand der Zeichnung erläutert. Es zeigt
Fig. 1 einen schematischen Querschnitt durch das Beduftungsgerät,
Fig. 1a eine Wechselkartusche,
Fig. 2 eine Außenansicht eines Beduftungsgeräts,
Fig. 3 einen Adapter zum Einsetzen des Beduftungsgeräts in einen Zigarettenanzünder,
Fig. 4 eine Andockstation für das Beduftungsgerät.

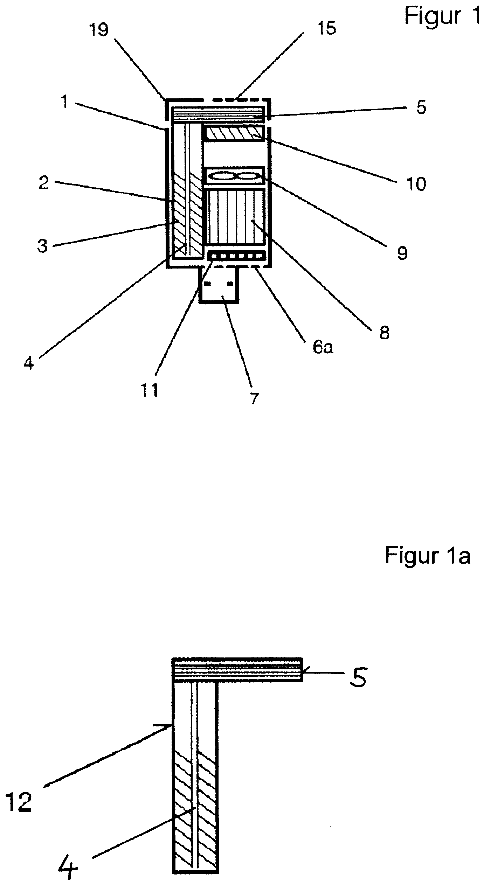

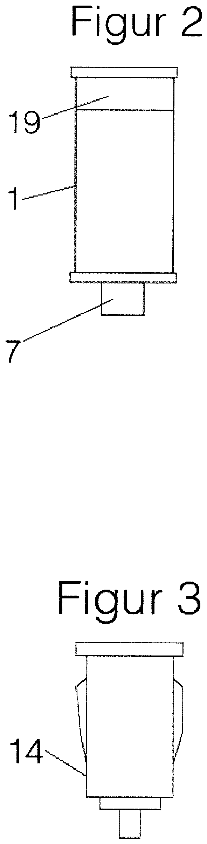

Fig. 1 zeigt ein Beduftungsgerät mit einem Gehäuse 1 mit einer abnehmbaren Kappe 19. Eine Außenansicht des Beduftungsgeräts ist in Fig. 2 dargestellt. Im Inneren des Gehäuses 1 befindet sich ein Duftstoffbehälter 2 aus einem gasundurchlässigen Werkstoff wie Glas oder Metall oder alternativ einem gasundurchlässig ausgekleideten Folienbeutel.

In den Duftstoffbehälter 2 ist flüssiger Duftstoff 3 eingefüllt, der mittels Kapillarkraft durch eine in dem Duftstoffbehälter 2 eintauchende Kapillarröhre 4 aus dem Behälter 2 nach oben ausströmen kann. Durch die Kapillarröhre 4 wird laufend Duftstoff nachgeliefert, abhängig vom Verbrauch. Am oberen Ende des Duftstoffbehälters 2 verläuft quer dazu ein Lamellenkörper 5, der aufgebaut ist aus einzelnen zueinander parallel verlaufenden Lamellen. Deren Abstände zueinander sind derart gering gehalten, dass dort der aus der Kapillarröhre 4 austretende Duftstoff in den Lamellenzwischenräumen weiter durch Kapillarwirkung verteilt über eine vergrößerte Oberfläche effektiv verdunstet wird.

Die Verdunstung wird verbessert durch ein elektrisch betriebenes Heizelement 10, beispielsweise aufgebaut aus Peletier- oder PTC-Bauteilen. Durch die Erwärmung der Umgebungsluft kommt es zu einer Konvektionsströmung zwischen Eintrittsöffnungen 6a auf der Gehäuseunterseite und Austrittsöffnungen 15 in der Kappe 19. In der erwärmten Luftströmung, die noch durch einen Mikroventilator 9 verstärkt wird, verdunstet der im Lamellenkörper 5 gespeicherte flüssige Duftstoff und gelangt mit der Luftströmung aus dem Inneren des Gehäuses 1 nach außen.

Im Inneren des Gehäuses 1 befindet sich ferner eine Regler-Platine 8, welche das Heizelement 10 und den Mikroventilator 9 über einen mechatronischen Regler 11 betätigt, mit dem Ergebnis, dass ein geregelter Duftluftstrom erwärmt und auf diese Weise eine dosierte Verdunstung des im Lamellenpaket 5 vorgehaltenen Duftstoffs erzielt wird. Der mit Duftstoff beladene Luftstrom gelangt durch die Austrittsöffnungen 15 in der Kappe 19 des Gehäuses 1 nach außen. Der Regler 11 gewährleistet somit eine geregelte Abgabe der gewünschten Duftluftmenge, welche aus dem Beduftungsgerät austritt, wobei der Luftdurchsatz über den Mikroventilator 9 und der Duftstoffanteil mittels des Heizelements 10 regelbar sind.

Der Duftstoffbehälter 2 mit der Kapillarröhre 4 bildet mit dem Lamellenkörper 5 eine fest verbundene Einheit in Art einer Wechselkartusche 12, die nach Abziehen der Kappe 19 entfernt und durch eine neue Kartusche ersetzt werden kann. Auf diese Weise wird sichergestellt, dass es zu keiner unerwünschten Vermischung verschiedener Duftstoffe kommt, denn jede neue Kartusche besitzt ein zugehöriges Lamellenpaket 5, welches nur von dem jeweils im Duftstoffbehälter 2 eingefüllten Duftstoff 3 benetzt wird.

Fig. 1a zeigt eine austauschbare Wechselkartusche 12 umfassend den Duftstoffbehälter 2 und den Lamellenkörper 5.

Ein USB-Gerätestecker 7, der auf der Unterseite des Gehäuses 1 angeordnet ist, versorgt das Beduftungsgerät mit elektrischer Energie, indem er mit einer externen Stromquelle verbunden wird. Als externe Stromquelle in einer Kraftfahrzeugpersonenzelle kommt eine mit der Autobatterie verbundene Steckdose, z.B. in Art des bekannten Zigarettenanzünders in Frage. Ein in Fig. 3 dargestellter USB-Stromadapter 14 stellt diese Verbindung zwischen dem USB-Gerätestecker 7 des Beduftungsgeräts und dem (nicht dargestellten) Zigarettenanzünder her.

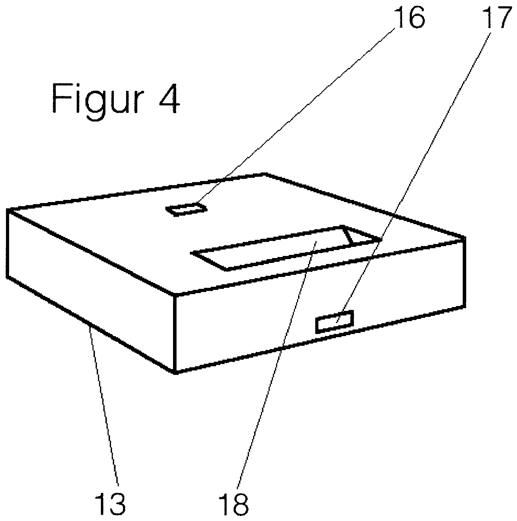

Als externe Stromquelle kommt auch eine Andockstation 13 in Frage, wie sie in Fig. 4 dargestellt ist. Die Andockstation besitzt auf ihrer Oberseite einen USB-Geräteeingang 16 zum Einstecken des USB-Gerätesteckers 7 des Beduftungsgeräts.

An ihrer Vorderseite besitzt die Andockstation 13 einen USB-Stromanschluss 17, der mit einer beliebigen Stromquelle wie einem Computer verbindbar ist. Im Inneren der Andockstation 13 ist ein (nicht dargestellter) Ventilator eingebaut, der an eine Ausblasöffnung 18 auf der Oberseite der Andockstation angeschlossen ist und auf diese Weise für eine zusätzliche Verteilung der aus dem Beduftungsgerät austretenden Duftluft sorgt.

## Patentansprüche

1. Beduftungsgerät für Innenräume, insbesondere Personenzellen in Kraftfahrzeugen, mit austauschbarem Duftstoffbehälter (2) für flüssigen Duftstoff und einem elektrischen Heizelement (10) zum Verdunsten von Duftstoff (3) vor dessen Ausströmen aus dem Beduftungsgerät, wobei mittels Kapillarkraft betriebene Fördermittel zur Entnahme von Duftstoff (3) aus dem Duftstoffbehälter (2) und zur Förderung des entnommenen Duftstoffs bis zum Heizelement vorgesehen sind,
wobei zur Entnahme von Duftstoff aus dem Duftstoffbehälter (2) wenigstens eine mit einem Ansaugende in den Duftstoffbehälter (2) eintauchende Kapillarröhre (4) vorgesehen ist, **dadurch gekennzeichnet, dass**
der Duftstoff (3) im Anschluss an deren Austrittsende einer dem Heizelement (10) zugeordneten Verteilungseinrichtung zugeführt wird,
die eine Lamellenanordnung (5) aufweist, deren Lamellenzwischenräume zur Verteilung des Duftstoffs (3) mittels Kapillarkraft geeignet ausgebildet sind.

2. Beduftungsgerät nach Anspruch 1,
**dadurch gekennzeichnet,**
**dass** zur Belüftung des Duftstoffs im Bereich der Verteilungseinrichtung eine gesonderte Ventilatoreinrichtung vorgesehen ist.

3. Beduftungsgerät nach Anspruch 2,
**dadurch gekennzeichnet,**
**dass** die Ventilatoreinrichtung als elektrischer Mikroventilator (9) ausgebildet ist.

4. Beduftungsgerät nach den Ansprüchen 1 und 2,
**dadurch gekennzeichnet,**
**dass** das Heizelement (10) zwischen Ventilatoreinrichtung und Verteilungseinrichtung angeordnet ist.

5. Beduftungsgerät nach Anspruch 1,
**dadurch gekennzeichnet,**
**dass** Duftstoffbehälter (2) mit Kapillarröhre (4) und Verteilungseinrichtung zusammen als austauschbare Wechselkartusche (12) ausgebildet sind.

6. Beduftungsgerät nach Anspruch 1,
**dadurch gekennzeichnet,**
**dass** dessen Bauteile im Inneren eines geschlossenen Gehäuses (1, 19) eingebaut sind, welches auf einander gegenüberliegenden Seiten Öffnungen (6a, 15) für den Eintritt von Umgebungsluft und für den Austritt von bedufteter Luft aufweist.

7. Beduftungsgerät nach Anspruch 6,
**dadurch gekennzeichnet,**
**dass** das Gehäuse (1) auf seiner der Austrittsöffnung (15) benachbarten Seite als abnehmbare Kappe (19) ausgebildet ist.

8. Beduftungsgerät nach Anspruch 7,
**dadurch gekennzeichnet,**
**dass** am Gehäuse (1) auf seiner der Kappe (19) gegenüberliegenden Seite ein USB-Gerätestecker (7) nach außen vorspringend angebaut ist.

9. Beduftungsgerät nach Anspruch 8,
**dadurch gekennzeichnet,**
**dass** sein USB-Gerätestecker (7) mittels eines USB-Stromadapters (14) mit einem in einem Kraftfahrzeug vorhandenen USB-Anschluss verbindbar ist.

10. Beduftungsgerät nach Anspruch 9,
**dadurch gekennzeichnet,**
**dass** der USB-Anschluss an einer Andockstation im Kraftfahrzeug ausgebildet ist.

11. Beduftungsgerät nach Anspruch 10,
**dadurch gekennzeichnet,**
**dass** die Andockstation durch den Zigarettenanzünder des Kraftfahrzeugs gebildet ist.

## Claims

1. Scenting device for interiors, in particular personal cells in motor vehicles, having an interchangeable scent container (2) for liquid scent and an electric heating element (10) for evaporating scent (3) before it flows out of the scenting device,
wherein conveying means operated by capillary force are provided for removing scent (3) from the scent container (2) and for conveying the removed scent to the heating element,
at least one capillary tube (4) which is immersed with a suction end in the scent container (2) is provided for removing scent from the scent container (2), **characterized in that** the scent (3) following its outlet end being supplied to a distribution device associated to the heating element (10), said distribution device having a lamella arrangement (5) whose lamella interspaces are designed to be suitable for distributing the scent (3) by means of capillary force.

2. Scenting device according to claim 1,
**characterized in that** a separate fan device is provided in the region of the distribution device for ventilating the scent.

3. Scenting device according to claim 2,
**characterized in that** the fan device is designed as an electric microfan (9).

4. Scenting device in accordance with claims 1 and 2,
**characterized in that** the heating element (10) is arranged between the fan device and the distribution device.

5. Scenting device according to claim 1,
**characterized in that** the scent container (2) with capillary tube (4) and distribution device are formed together as interchangeable cartridge (12).

6. Scenting device according to claim 1,
**characterized in that** its components are installed inside a closed housing (1, 19) which has openings (6a, 15) on opposite sides for the inlet of ambient air and for the outlet of scented air.

7. Scenting device according to claim 6,
**characterized in that** the housing (1) is designed as a removable cap (19) on its side adjacent to the outlet opening (15).

8. Scenting device according to claim 7,
**characterized in that** a USB device plug (7) is mounted on the housing (1) on its side opposite the cap (19) so as to project outwards.

9. Scenting device according to claim 8,
**characterized in that** its USB device plug (7) can be connected by means of a USB power adapter (14) to a USB connection present in a motor vehicle.

10. Scenting device according to claim 9,
**characterized in that** the USB port is formed at a docking station in the motor vehicle.

11. Scenting device according to claim 10,
**characterized in that** the docking station is formed by the cigarette lighter of the motor vehicle.

## Revendications

1. Dispositif de parfumage d'intérieur, en particulier de cellules personnelles dans des véhicules automobiles, comprenant un récipient (2) de parfum interchangeable pour de parfum liquide et un élément chauffant électrique (10) pour évaporer le parfum (3) avant qu'il s'échappe du dispositif de parfumage,
dans lequel des moyens de transport actionnés au moyen d'une force capillaire sont prévus pour enlever le parfum (3) du récipient (2) de parfum et pour transporter le parfum enlevé à l'élément chauffant,
et dans laquelle au moins un tube capillaire (4) qui est immergé avec une extrémité d'aspiration dans le récipient (2) de parfum est prévu pour enlever le parfum du récipient (2) de parfum, lequel dispositif se **caractérise par**
en ce que le parfum (3) est fourni après l'extrémité de sortie du tube capillaire (4) à un dispositif de distribution associé à l'élément chauffant (10),
qui présente un agencement de lamelles (5) dont les espaces lamellaires sont conçus pour la diffusion du parfum (3) au moyen d'une force capillaire.

2. Dispositif de parfumage selon la revendication 1,
se **caractérise par**
en ce qu'un dispositif de ventilation séparé est prévu dans la zone du dispositif de distribution pour ventiler le parfum.

3. Dispositif de parfumage selon la revendication 2,
se **caractérise par**
en ce que le dispositif de ventilation est conçu comme un microventilateur électrique (9).

4. Dispositif de parfumage selon les revendications 1 et 2,
se **caractérise par**
en ce que l'élément chauffant (10) est disposé entre l'appareil de ventilation et l'appareil de distribution.

5. Dispositif parfumant selon la revendication 1,
se **caractérise par**
en ce que le récipient (2) de parfum avec tube capillaire (4) et dispositif de distribution sont conçus ensemble comme cartouche interchangeable (12).

6. Dispositif de parfumage selon la revendication 1,
se **caractérise par**
en ce que ses composants sont installés à l'intérieur d'un boîtier fermé (1, 19) qui présente des ouvertures (6a, 15) sur des côtés opposés pour l'entrée de l'air ambiant et pour la sortie de l'air parfumé.

7. Dispositif parfumant selon la revendication 6,
se **caractérise par**
en ce que le boîtier (1) est réalisé sous la forme d'un capuchon amovible (19) sur son côté adjacent à l'ouverture de sortie (15).

8. Dispositif parfumant selon la revendication 7,
se **caractérise par**
en ce qu'un connecteur de dispositif USB (7) est monté sur le boîtier (1) sur son côté opposé au capuchon (19) de manière à faire saillie vers l'extérieur.

9. Dispositif de parfumage selon la revendication 8,
se **caractérise par**
en ce que sa fiche de dispositif USB (7) peut être connectée au moyen d'un adaptateur d'alimentation USB (14) à une connexion USB présente dans un véhicule automobile.

10. Dispositif parfumant selon la revendication 9,
se **caractérise par**
en ce que la connexion USB est formée à une station d'accueil dans le véhicule automobile.

11. Dispositif parfumant selon la revendication 10,
se **caractérise par**
que la station d'accueil est formée par l'allume-cigare du véhicule automobile.
